# EUROPÄISCHE PATENTSCHRIFT

(11) **EP 2 413 788 B1**
(45) Veröffentlichungstag und Bekanntmachung des Hinweises auf die Patenterteilung: **23.12.2020**
(21) Anmeldenummer: 10721288.8
(22) Anmeldetag: 31.03.2010
(51) Int. Cl.: A61B 5/024, A61B 5/22, A63B 22/00

(54) **VERFAHREN ZUR BESTIMMUNG DER ALLGEMEINEN FITNESS EINES PROBANDEN**
METHOD FOR DETERMINING THE GENERAL FITNESS OF A TEST SUBJECT
PROCÉDÉ DESTINÉ À DÉTERMINER LA FORME PHYSIQUE GÉNÉRALE D'UN SUJET DE TEST

(30) Priorität: 01.04.2009 DE 102009015273
(43) Veröffentlichungstag der Anmeldung: 08.02.2012
(73) Patentinhaber: adidas AG, 91074 Herzogenaurach (DE)
(72) Erfinder: RÖCKER, Kai, 79102 Freiburg (DE); GOLLHOFER, Albert, 79252 Stegen (DE)
(74) Vertreter: Wegner, Hans
(86) Internationale Anmeldenummer: PCT/DE2010/000371
(87) Internationale Veröffentlichungsnummer: WO 2010/112010

(56) Entgegenhaltungen:
- GB-A- 2 187 554
- US-A- 4 830 021
- US-A1- 2002 198 463
- US-A1- 2008 033 311

## Beschreibung

### Stand der Technik

Die Erfindung geht aus von einem Verfahren zur Bestimmung der allgemeinen Fitness eines Probanden.

Die allgemeine Fitness eines menschlichen Probanden wird durch seine Ausdauerleistungsfähigkeit bestimmt. Die Ausdauerleistungsfähigkeit hängt von verschiedenen physiologischen Faktoren ab. Hierzu zählen beispielsweise Alter, Geschlecht, Größe, Gewicht, Körperfett und Trainingsumfang eines Probanden. Die Ausdauerleistungsfähigkeit kann beispielsweise aus der maximalen Herzfrequenz, der Ausgangsherzfrequenz und/ oder der Anstiegscharakteristik der Herzfrequenz bei einer körperlichen Belastung bestimmt werden. Im Zusammenhang mit der Leistungsdiagnostik ist die möglichst exakte Bestimmung der Ausdauerleistungsfähigkeit von großem Interesse. Die Ausdauerleistungsfähigkeit ist zum Beispiel ein wichtiger prognostischer Faktor für das Risiko, eine Herz-Kreislauferkrankung zu erleiden. Darüber hinaus ist die Ausdauerleistungsfähigkeit ein gutes Maß für die Fähigkeit, alltägliche körperliche Anforderungen zu meistern. Zudem kann anhand der Ausdauerleistungsfähigkeit der Erfolg eines sportlichen oder präventiven Ausdauertrainings optimiert und objektiviert werden. So ermöglicht die exakte Messung der Ausdauerleistungsfähigkeit in der Leistungsdiagnostik zum Beispiel die Erstellung von Trainingsempfehlungen für den Freizeitsport, die Trainingssteuerung im Ausdauersport, die Trainingsverordnung in der Rehabilitation, objektive Leistungsprognosen für den Wettkampfsport, die Feststellung oder Verlaufsbeurteilung einer funktionellen Beeinträchtigung bei schweren chronischen internistischen Erkrankungen zum Beispiel Herzinsuffizienz, die präoperative Evaluation bei speziellen Erkrankungen zum Beispiel zur Frage der Herztransplantation oder bei Lungenteilresektion, die arbeitsmedizinische Evaluation und den Präventionssport.

Aus der Leistungsdiagnostik sind Verfahren zur Bestimmung der Ausdauerleistungsfähigkeit bekannt, bei denen der Proband einem Belastungstest unterzogen wird, der in seiner Form und Dauer mit der Belastung übereinstimmt, für die eine Aussage getroffen werden soll. Zum Beispiel erfolgt für die Messung der Leistungsfähigkeit im Langstreckenlauf ein ca. 30minütiger Test mit ansteigender Belastungsintensität auf dem Laufband. Bei einem derartigen Test werden neben der physikalischen Leistung beispielsweise auch die Herzfrequenz und die Laktatkonzentration im Blut bestimmt. Aus dem zeitlichen Verlauf dieser Messgrößen kann mit hoher Genauigkeit eine Aussage zur Leistungsfähigkeit im Langstreckenlauf getroffen werden. Entsprechende Belastungstests sind für weitere Bereiche bekannt. Hierzu werden beispielsweise Fahrrad-Ergometer, Ruder-Ergometer, Kanu-Ergometer, Handbike-Ergometer und Handkurbel-Ergometer eingesetzt. Als Referenzgröße für die Ausdauerleistungsfähigkeit dient häufig die so genannte anaerobe Schwelle. Dieser Wert kann aus den Ergebnissen einer Leistungsdiagnostik mit unterschiedlichen Methoden ermittelt werden.

In der US 2008/033311 A1 sind ein Verfahren und eine Vorrichtung zur Bestimmung der Herzfrequenz und der Leistung während einer körperlichen Belastung, wobei wird die Leistung schrittweise in mehreren Zeitintervallen gesteigert und die Herzfrequenz erfasst wird. Während zunächst der zeitliche Verlauf der Leistung und der Herzfrequenz parallel ist, entwickelt sich ab einem bestimmten Punkt die Herzfrequenz anders als die Leistung. Die Leistung bleibt konstant. Die Herzfrequenz steigt an. Dies gibt Aufschluss über die anaerobe Schwelle. Der Test wird beendet, wenn die maximale Steigerung erreicht ist.

Aus der GB 2 187 554 A ist eine Vorrichtung zum Körpertraining bekannt, welche mit einem Detektor zur Erfassung physiologischer Parameter insbesondere der Pulsrate und der Leistung, ausgestattet ist. Während eines Körpertrainings werden diese Parameter kontinuierlich erfasst, um das Training zu optimieren und um einen gewünschten Trainingserfolg zu erzielen.

In der US 2002/198463 A1 sind eine Vorrichtung und ein Verfahren zur Erfassung und Modifizierung der physiologischen Kondition eines Individuums offenbart. Dabei werden Zvklusparameter aus einer Aufzeichnung des zeitlichen Verlaufs von Herzfrequenzdaten gewonnen, welche während des Trainings eines Probanden erfasst werden. Das Training erfolgt in Intervallen. Dabei wechseln sich Phasen der körperlichen Belastung mit Entspannungsphasen ab. Das Training erfolgt über mehrere Tage.

Die US 4 830 021 A betrifft ein System zum Überwachen menschlicher Bewegungsaktivitäten über lange Zeiträume durch Aufzeichnen der Beschleunigung eines Probanden während solcher Zeiträume und anschließendes Umwandeln der Beschleunigung in Fuß- / Bodenkräfte des jeweiligen Probanden durch eine zuvor kalibrierte Umrechnungstabelle, welche sich auf den jeweiligen Probanden bezieht.

Die bekannten Verfahren zur Bestimmung der allgemeinen Fitness eines Probanden weisen den Nachteil auf, dass sie zeitlich und apparativ sehr aufwendig sind und mit einer maximalen körperlichen Beanspruchung des Probanden verbunden sind. Diese hohe körperliche Beanspruchung sowie der hohe Kosten- und Personalaufwand zur Durchführung der bekannten Verfahren haben bisher eine breite Anwendung beispielsweise in Fitness- und Sportstudios sowie in Sportgeschäften verhindert. Es sind zwar auch Verfahren bekannt, bei denen die Ausdauerleistungsfähigkeit ohne eine körperliche Belastung bestimmt wird, jedoch führen diese zu ungenauen Ergebnissen.

Der Erfindung liegt die Aufgabe zugrunde, ein Verfahren zur Bestimmung der allgemeinen Fitness eines Probanden zur Verfügung zu stellen, welches zu einem exakten und reproduzierbaren Ergebnis führt, ohne den Probanden einer hohen körperlichen Belastung über längere Zeit auszusetzen, und ohne dabei auf einen hohen apparativen und zeitlichen Aufwand angewiesen zu sein.

### Die Erfindung und ihre Vorteile

Die Aufgabe wird durch ein Verfahren mit den Merkmalen des Anspruchs 1 gelöst. Das erfindungsgemäße Verfahren zeichnet sich dadurch aus, dass der qualitative zeitliche Verlauf der Herzfrequenz bei einer konstanten körperlichen Belastung als mathematische Funktion mit mindestens einem von dem Probanden abhängigen Parameter vorgegeben wird. Dabei wird ausgenutzt, dass die Herzfrequenz bei plötzlich einsetzender körperlicher Belastung eine typische Verlaufscharakteristik aufzeigt, die bekannt ist. Der zeitliche Verlauf der Herzfrequenz ist charakterisiert durch einen ersten Abschnitt mit zunächst langsamen Anstieg zu Beginn einer körperlichen Belastung, einem sich daran anschließenden zweiten Abschnitt mit schnellem Anstieg bei Fortdauer der körperlichen Belastung und einem sich daran anschließenden dritten Bereich mit abnehmendem Anstieg der Herzfrequenz. Der zweite Abschnitt beginnt typischerweise nach 3 bis 5 Sekunden der körperlichen Belastung. Im dritten Abschnitt nähert sich die Herzfrequenz einem stabilen Wert der Herzfrequenz an, wobei sich dieser Wert innerhalb einer vorgegebenen Messgenauigkeit bei andauernder konstanter körperlicher Belastung nicht mehr ändert. Er wird als maximale Herzfrequenz dieser Belastungsform bezeichnet. Dieser Wert wird typischerweise nach 2 bis 3 Minuten eines Körpertrainings erreicht. Aufgrund der zunächst langsamen Anstiegs, einem sich daran anschließenden Abschnitt mit schnellem Anstieg und einer Annäherung an einen Maximalwert gleicht der Verlauf der Funktion qualitativ einer Hyperbel. Die Zeitintervalle der ersten, zweiten und dritten Abschnitte sowie die maximalen und minimalen Werte der Herzfrequenz sind von Proband zu Proband verschieden und hängen darüber hinaus von der Belastungsintensität und der Belastungsdauer ab. Der qualitative zeitliche Verlauf der Herzfrequenz ist jedoch für alle Probanden und alle Arten von körperlichen Belastungen gleich. Die Parameter dieser Funktion werden durch Messen der Herzfrequenz eines Probanden unter konstanter körperlicher Belastung bestimmt. Hierfür genügt typischer Weise eine konstante körperliche Belastung während einer Dauer von 5 bis maximal 50 Sekunden. Für die meisten Probanden ist eine körperliche Belastung während einer zeitlichen Dauer von 20 bis 30 Sekunden ausreichend, um die Parameter der mathematischen Funktion mit hoher Genauigkeit zu bestimmen. Sind die Parameter der Funktion ermittelt, so kann die maximale Herzfrequenz durch numerische Verfahren, nämlich Extrapolation, bestimmt werden. Zur Bestimmung der Parameter aus den Messwerten der Herzfrequenz eignen sich beispielsweise nichtlineare Verfahren zur Parameteroptimierung. Hierzu zählt beispielsweise ein so genanntes Kurvenfitting unter Anwendung des Marquard-Levenberg-Algorithmus.

Die Ausgangsherzfrequenz entspricht der Herzfrequenz eines Probanden in Ruhe ohne körperliche Belastung. Häufig hat der Proband bereits zu Beginn der Messung seiner Herzfrequenz eine gegenüber der Ausgangsherzfrequenz erhöhte Herzfrequenz, da er sich bereits zuvor, wenn auch nur wenig, bewegt hat, oder er aufgeregt ist. Die Ausgangsherzfrequenz kann aus der mit den ermittelten Parametern versehenen Funktion durch numerische Verfahren, nämlich durch Extrapolation bestimmt werden.

Aus der mit den Parametern versehenen Funktion kann darüber hinaus die Anstiegscharakteristik der Herzfrequenz über der Zeit bestimmt werden. Hierzu zählt beispielsweise die maximale Steigung der Funktion. Sie gibt genau wie die maximale Herzfrequenz und die Ausgangsherzfrequenz Aufschluss über die allgemeine Fitness insbesondere über die Ausdauerleistungsfähigkeit eines Probanden.

Die maximale Herzfrequenz, die Ausgangsherzfrequenz und die Anstiegscharakteristik können alternativ oder kumulativ bestimmt werden. Es können auch zwei der Größen bestimmt werden.

Zur Bestimmung der allgemeinen Fitness eines Probanden genügt es, dass sich der Proband innerhalb eines kurzen Zeitintervalls von weniger als 50 Sekunden einer konstanten körperlichen Belastung unterzieht und während dieser Dauer die Herzfrequenz des Probanden kontinuierlich gemessen wird. Hierzu führt der Proband eine körperliche Bewegung mit kardiopulmonaler Beanspruchung aus. Der Proband muss weder bei seiner Ausgangsherzfrequenz mit der körperlichen Belastung beginnen noch muss er sich dieser bis zum Erreichen der maximalen Herzfrequenz unterziehen. Bezogen auf die oben angegebenen drei Abschnitte des Anstiegs der Herzfrequenz bei körperlicher Belastung werden die Messwerte der Herzfrequenz bevorzugt im zweiten Abschnitt bestimmt. Die für die Bestimmung der allgemeinen Fitness notwendigen Werte der Herzfrequenz werden gemessen, bevor der Proband zu schwitzen beginnt. Das erfindungsgemäße Verfahren kann daher auch in Sportgeschäften und bei Informationsveranstaltungen angewandt werden.

Die Bestimmung der maximalen Herzfrequenz und/ oder der Ausgangsherzfrequenz und/ oder der Anstiegscharakteristik der Herzfrequenz erfolgt aus den Messwerten der Herzfrequenz durch mathematische Berechnungen. Hierzu kann ein Rechner verwendet werden. Das erfindungsgemäße Verfahren zeichnet sich damit durch einen geringen apparativen und zeitlichen Aufwand aus. Darüber hinaus muss sich der Proband lediglich einer kurzen körperlichen Belastung unterziehen. Hierfür eignen sich beispielsweise Kniehebellauf, HockStreck-Sprünge, Fahrradfahren auf einem Ergometer, Laufen auf einem Laufband oder einem Stepper oder die Anwendung sonstiger Ergometer. Die Belastung ist aufgrund der kurzen Dauer von typischerweise weniger als 60 sec so gering, dass sich der Proband dabei nicht körperlich verausgabt.

Da die Bestimmung der maximalen Herzfrequenz anhand eines körperlichen Belastungstests des Probanden erfolgt, liefert das Verfahren exakte und auf den Probanden abgestimmte Werte.

Die Ausdauerleistungsfähigkeit und andere trainingsrelevante Größen, wie beispielsweise eine Trainingsempfehlung für ein Körpertraining des Probanden können anhand der maximalen Herzfrequenz und/ oder der Ausgangsherzfrequenz und/ oder der Anstiegscharakteristik rechnerisch bestimmt werden. Hierzu eignet sich beispielsweise eine multivariante Modellrechnung. Über mathematisch-statistische Verfahren lassen sich aus einem Satz möglicher Einflussgrößen diejenigen herausfiltern, die eine signifikante prädiktive Potenz für eine gewünschte Zielgröße aufweisen. Ein derartiges Verfahren ist beispielsweise die Stepwise Multiple Regression. Das erfindungsgemäße Verfahren ermöglicht beispielsweise die Bestimmung der Leistung an der anaeroben Schwelle mit hoher Genauigkeit. Zur Erzielung exakter und reproduzierbarer Werte für die Ausdauerleistungsfähigkeit können weitere physiologische Faktoren eines Probanden wie beispielsweise Alter, Geschlecht, Körpergröße, Gewicht, Körperfett, Bauchumfang oder Oberschenkelumfang berücksichtigt werden. Durch die Berücksichtigung dieser anamnetischen Informationen kann die Genauigkeit der aus den Messwerten abgeleiteten Ausdauerleistungsfähigkeit erhöht werden.

Neben der Bestimmung der Ausdauerleistungsfähigkeit können mit dem erfindungsgemäßen Verfahren auch zusätzliche Informationen für eine Trainingsempfehlung eines Probanden ermittelt werden. Beispielsweise kann bestimmt werden, wie hoch die Herzfrequenz für ein optimales Ausdauertraining ansteigen sollte. Darüber hinaus kann eine Empfehlung für die Leistung während des Körpertrainings angegeben werden. Beim einem Lauftraining wird hierzu beispielsweise die Geschwindigkeit angegeben, mit der der Proband sich fortbewegen sollte. Des weiteren kann der Effekt eines Ausdauertrainings aus der Relation zwischen Leistung und maximaler Herzfrequenz ermittelt werden. Hierzu muss zusätzlich zu der Herzfrequenz die Leistung während der körperlichen Belastung bestimmt werden.

Nach einer vorteilhaften Ausgestaltung der Erfindung kann zur Verbesserung des Ergebnisses eine fortlaufende rechnerische Plausibilitätsprüfung erfolgen. Auf der Grundlage dieser Plausibilitätsprüfung kann die körperliche Belastung beispielsweise zu einem früheren Zeitpunkt abgebrochen oder auch um eine gewisse Dauer verlängert werden. Dies ist zum Beispiel der Fall, wenn die Bestimmung der Parameter der mathematischen Funktion nach kurzer Zeit abgeschlossen ist, oder wenn die Messung aus Qualitätsgründen wiederholt werden muss. Hierüber kann der Proband mit einer optischen Anzeigeeinrichtung informiert werden.

Nach einer weiteren vorteilhaften Ausgestaltung des erfindungsgemäßen Verfahren wird zusätzlich die Leistung des Probanden während der körperlichen Belastung bestimmt. Bei einer körperlichen Belastung in Form eines Kniehebellaufs oder bei Hockstreck-Sprüngen kann die Leistung zum Beispiel durch eine Messplatte für Bodenreaktionskräfte bestimmt werden. Aus der Schrittfrequenz und der auf die Messplatte ausgeübten Kräfte wird die Leistung berechnet. Unterzieht sich der Proband einer körperlichen Belastung auf einem Ergometer, so kann die Leistung mittels in das Ergometer integrierter Detektoren für die Repetitionsrate und/ oder die Kraft bestimmt werden. Anhand der maximalen Herzfrequenz in Relation zu der zugehörigen Leistung kann die Ausdauerleistungsfähigkeit noch besser und exakter bestimmt werden. Ferner ist die Feststellung weiterer trainingsrelevanter Größen möglich. Hierzu zählt beispielsweise die Herzfrequenz des Probanden für ein optimales Ausdauertraining.

Nach einer weiteren vorteilhaften Ausgestaltung des erfindungsgemäßen Verfahrens wird die Leistung während des Zeitintervalls überwacht und innerhalb vorgegebener Grenzen konstant gehalten. Eine körperliche Belastung mit konstanter Leistung während der Messung der Herzfrequenz hat den Vorteil, dass die maximale Herzfrequenz mit hoher Genauigkeit bestimmt werden kann.

Nach einer weiteren vorteilhaften Ausgestaltung des erfindungsgemäßen Verfahrens wird der Proband über eine Einhaltung der konstanten Leistung und/ oder über eine Abweichung von der konstanten Leistung informiert. Er wird damit während der körperlichen Belastung dazu motiviert und angehalten, sich mit konstanter Leistung zu bewegen.

Nach einer weiteren vorteilhaften Ausgestaltung des erfindungsgemäßen Verfahrens Verfahren wird die Ausdauerleistungsfähigkeit aus der maximalen Herzfrequenz und/ oder der Ausgangsherzfrequenz und/ oder der Anstiegscharakteristik der Herzfrequenz bestimmt. Hierbei kann die Leistung des Probanden während der körperlichen Belastung berücksichtigt werden. Zur Bestimmung der Ausdauerleistungsfähigkeit eignet sich beispielsweise eine multivariante Modellrechnung und/ oder durch ein multifaktorielles Prognosemodell.

Nach einer weiteren vorteilhaften Ausgestaltung des erfindungsgemäßen Verfahrens werden die Parameter der Funktion zu jedem ermittelten Herzschlag des Probanden bestimmt. Dies ermöglicht eine besonders schnelle Berechnung der Parameter.

Nach einer weiteren vorteilhaften Ausgestaltung des erfindungsgemäßen Verfahrens wird die Messung der Herzfrequenz beendet, wenn sich die aus mindestens zwei aufeinander folgenden Herzschlägen bestimmten Parameter um weniger als einen vorgegebenen Wert unterscheiden. Darüber hinaus kann die Messung der Herzfrequenz unter körperlicher Belastung beispielsweise fortgesetzt werden, solange die Steigung der zugehörigen Herzfrequenz-ZeitFunktion zunimmt. Sie kann beispielsweise beendet werden, sobald die Steigung abnimmt.

Nach einer weiteren vorteilhaften Ausgestaltung des erfindungsgemäßen Verfahrens wird der qualitative zeitliche Verlauf der Herzfrequenz als mathematische Funktion aus dem zeitlichen Verlauf der Herzfrequenz einer Vielzahl von Probanden unter körperlicher Belastung bestimmt.

Nach einer weiteren vorteilhaften Ausgestaltung des erfindungsgemäßen Verfahrens wird während der körperlichen Belastung der überwiegende Teil der Muskelmasse des Probanden dynamisch und konstant belastet. Hierzu wird der Proband einer körperlichen Belastung unterzogen, bei der die Muskeln der Arme, Beine und des Oberkörpers betätigt werden. So können beispielsweise zum Kniehebellauf zusätzlich Gewichte an den Händen getragen und bewegt werden. Bei einer körperlichen Belastung der gesamten Muskelmasse ist der Bedarf an zu verstoffwechselndem Sauerstoff hoch, was zu einem sehr genauen Ergebnis der maximalen Herzfrequenz führt.

Eine Vorrichtung zur Ausführung des Vefahrens zeichnet sich dadurch aus, dass sie ausgestattet ist mit einer Einrichtung zur Messung der Herzfrequenz des Probanden, mit einer Speichereinrichtung um eine mathematische Funktion für den zeitlichen Verlauf der Herzfrequenz unter körperlicher Belastung abzuspeichern und mit einer Recheneinrichtung um die Parameter der mathematischen Funktion aus den Messwerten der Herzfrequenz zu berechnen und die maximale Herzfrequenz und/ oder die Ausgangsherzfrequenz ohne körperliche Belastung und/ oder die Anstiegscharakteristik der Herzfrequenz aus der mit den berechneten Parametern versehenen Funktion numerisch zu bestimmen. Hierzu eignet sich beispielsweise ein Computer.

Nach einer vorteilhaften Ausgestaltung der Vorrichtung ist diese mit einer Einrichtung zur Ausführung einer körperlichen Bewegung mit kardiopulmonaler Beanspruchung eines Probanden ausgestattet. Dabei kann es sich beispielsweise um ein Fahrrad-Ergometer, ein Ruder-Ergometer, ein Kanu-Ergometer, ein Handbike-Ergometer, ein Handkurbel-Ergometer oder ein Laufband handeln.

Nach einer vorteilhaften Ausgestaltung der Vorrichtung ist diese mit einer Einrichtung zur Bestimmung der bei der Bewegung erzeugten Leistung ausgestattet.

Nach einer vorteilhaften Ausgestaltung der Vorrichtung ist diese mit einer Messplatte für Bodenreaktionskräfte ausgestattet.

Weitere Vorteile und vorteilhafte Ausgestaltungen sind der nachfolgenden Beschreibung, der Zeichnung und den Ansprüchen zu entnehmen.

### Zeichnung

In der Zeichnung sind schematisch die Verfahrensschritte dargestellt. Es zeigen:
- Figur 1: Flussdiagramm des Verfahrens zur Bestimmung der Ausdauerleistungsfähigkeit,
- Figur 2: typischer zeitlicher Verlauf der Herzfrequenz unter körperlicher Belastung,
- Figur 3: schematische Darstellung der Datenerfassung und der Bestimmung der allgemeinen Fitness eines Probanden.

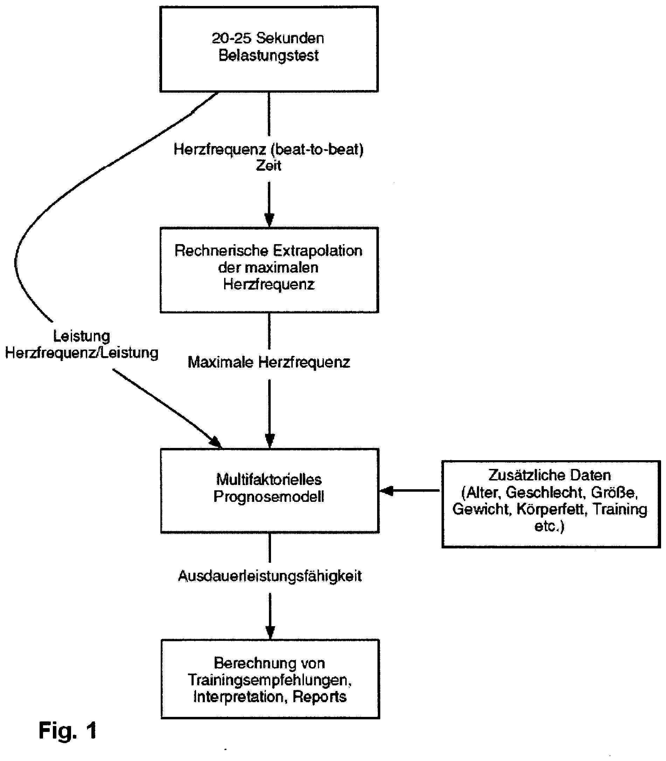

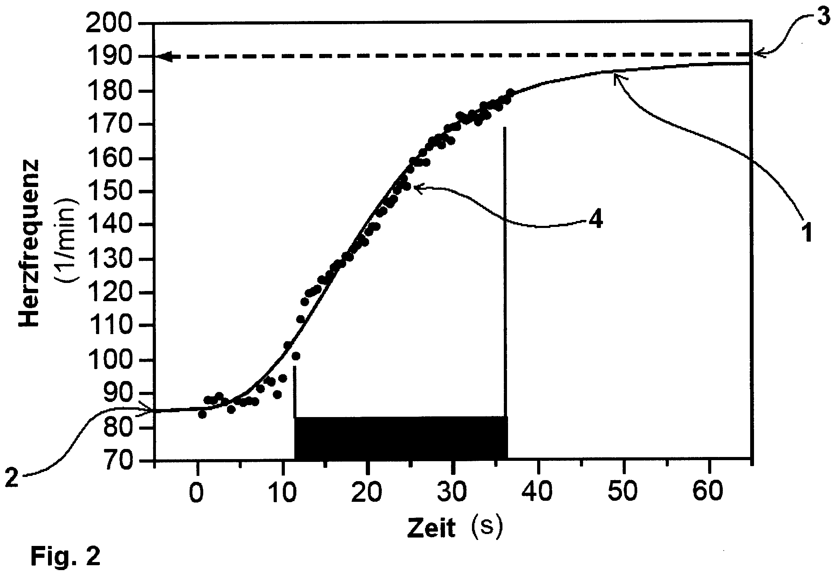

Bei dem in Figur 1 schematisch aufgezeigten Verfahren unterzieht sich ein Proband einem Belastungstest während einer Dauer 20 bis 25 Sekunden. Hierzu führt der Proband mit oder ohne Trainingsgerät eine Bewegung mit kardiopulmonaler Beanspruchung aus. Während des Belastungstests wird die Herzfrequenz ständig gemessen, so dass jeder Herzschlag erfasst wird. Durch rechnerische Extrapolation wird anhand der Messwerte und einer in Figur 2 dargestellten mathematischen Funktion die maximale Herzfrequenz des Probanden bestimmt. Parallel dazu wird während des Belastungstests die Leistung des Probanden ermittelt. Es kann auch die Relation aus Herzfrequenz und Leistung bestimmt werden. Diese fließen zusammen mit der maximalen Herzfrequenz in ein multifunktionales Prognosemodell. Ferner werden weitere physiologische Faktoren des Probanden eingegeben wie Alter, Geschlecht, Körpergröße, Gewicht, Körperfett und Training des Probanden. Aus allen eingegebenen Größen wird die Ausdauerleistungsfähigkeit bestimmt. Diese wird für die Berechnung von Trainingsempfehlungen, Interpretationen und Reports des Probanden benutzt.

In Figur 2 ist exemplarisch für einen Probanden die mathematische Funktion der Herzfrequenz über der Zeit bei einer körperlichen Belastung dargestellt. Die zugehörige Kurve 1 beginnt bei einer Ausgangsherzfrequenz 2, die der Proband in Ruhe ohne körperliche Belastung und ohne geistige Anspannung aufweist. In einem ersten Abschnitt bis 5 sec steigt die Herzfrequenz nur wenig an. In einem zweiten Abschnitt von 5 sec bis 35 sec nimmt die Herzfrequenz stark zu. In einem dritten Abschnitt ab 35 sec nimmt der Anstieg ab. Die Herzfrequenz nähert sich der maximalen Herzfrequenz 3 an. Der Proband beginnt zum Zeitpunkt 0 sec mit einem Belastungstraining. Von 12 sec bis 35 sec wird die Herzfrequenz gemessen. Danach kann sowohl die Messung als auch das Belastungstraining beendet werden. Die Messwerte der Herzfrequenz sind durch Punkte 4 dargestellt. Aus den Messwerten wird durch Kurvenfitting die Kurve bestimmt. Ferner werden die Parameter der zugehörigen mathematischen Funktion bestimmt.

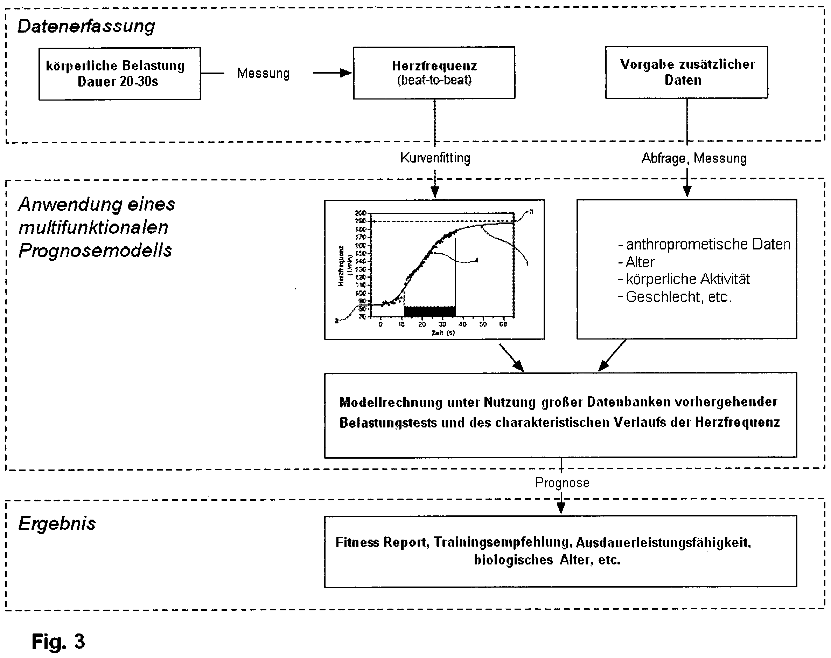

In Figur 3 sind die Datenerfassung und die Bestimmung der allgemeinen Fitness eines Probanden schematisch dargestellt. Als Daten werden die Herzfrequenz unter körperlicher Belastung während 20 bis 30 sec gemessen und physische Daten des Probanden abgefragt. Aus den Messdaten der Herzfrequenz und dem qualitativ vorgegebenen zeitlichen Verlauf der Herzfrequenz wird mittels Kurvenfitting die zugehörige mathematische Funktion bestimmt. Das dargestellte Schaubild entspricht Figur 2. Aus der Funktion und den abgefragten Daten des Probanden werden mittels Modellrechnung die Ausdauerleistungsfähigkeit, ein Bericht betreffend die Fitness, eine Trainingsempfehlung und das biologische Alter des Probanden bestimmt. Hierbei handelt es sich um Näherungswerte, die eine hohe Genauigkeit aufweisen.

## Patentansprüche

1. Verfahren zur Bestimmung der allgemeinen Fitness eines Probanden geumfassend folgende Verfahrensschritte:
Vorgeben des qualitativen zeitlichen Verlaufs der Herzfrequenz bei einer konstanten körperlichen Belastung eines Probanden als mathematische Funktion mit mindestens einem von einem Probanden abhängigen Parameter,
Messen der Herzfrequenz des Probanden unter kontinuierlicher körperlicher Belastung während eines Zeitintervalls, wobei sich der Proband innerhalb dieses kurzen Zeitintervalls von weniger als 50 Sekunden einer konstanten körperlichen Belastung unterzieht und während dieser Dauer die Herzfrequenz des Probanden kontinuierlich gemessen wird, Bestimmen, mit einer Recheneinrichtung, des oder der Parameter der mathematischen Funktion aus den Messwerten, und **gekennzeichnet durch**: Bestimmen, mit der Recheneinrichtung, der maximalen Herzfrequenz aus der Funktion und den Messwerten durch ein numerisches Verfahren, nämlich durch Extrapolation, und/ oder Bestimmen, mit der Recheneinrichtung, der Ausgangsherzfrequenz ohne körperliche Belastung aus der Funktion und den Messwerten durch ein numerisches Verfahren, nämlich durch Extrapolation.

2. Verfahren nach Anspruch 1, **dadurch gekennzeichnet, dass** zusätzlich die Leistung des Probanden während der körperlichen Belastung bestimmt wird.

3. Verfahren nach Anspruch 2, **dadurch gekennzeichnet, dass** die Leistung während des Zeitintervalls überwacht und innerhalb vorgegebener Grenzen konstant gehalten wird.

4. Verfahren nach Anspruch 3, **dadurch gekennzeichnet, dass** der Proband über eine Einhaltung der konstanten Leistung und/ oder über eine Abweichung von der konstanten Leistung informiert wird.

5. Verfahren nach einem der vorhergehenden Ansprüche, **dadurch gekennzeichnet, dass** die Ausdauerleistungsfähigkeit aus der maximalen Herzfrequenz und/ oder der Ausgangsherzfrequenz und/ oder der Anstiegscharakteristik der Herzfrequenz bestimmt wird.

6. Verfahren nach einem der vorhergehenden Ansprüche, **dadurch gekennzeichnet, dass** aus der maximalen Herzfrequenz und/ oder der Anstiegscharakteristik der Herzfrequenz und/ oder der Ausgangsherzfrequenz eine Trainingsempfehlung für den Probanden bestimmt wird.

7. Verfahren nach Anspruch 5 oder 6, **dadurch gekennzeichnet, dass** physische Merkmale des Probanden vorgegebenen werden und bei der Bestimmung der Ausdauerleistungsfähigkeit und/ oder bei der Bestimmung einer Trainingsempfehlung berücksichtigt wird.

8. Verfahren nach einem der vorhergehenden Ansprüche, **dadurch gekennzeichnet, dass** die Parameter der Funktion zu jedem ermittelten Herzschlag des Probanden bestimmt werden.

9. Verfahren nach Anspruch 8, **dadurch gekennzeichnet, dass** die Messung der Herzfrequenz beendet wird, wenn sich die aus mindestens zwei aufeinanderfolgenden Herzschlägen bestimmten Parameter um weniger als einen vorgegebenen Wert unterscheiden.

10. Verfahren nach einem der vorhergehenden Ansprüche, **dadurch gekennzeichnet, dass** der qualitative zeitliche Verlauf der Herzfrequenz als mathematische Funktion aus dem zeitlichen Verlauf der Herzfrequenz einer Vielzahl von Probanden unter körperliche Belastung bestimmt wird.

11. Verfahren nach einem der vorhergehenden Ansprüche, **dadurch gekennzeichnet, dass** während der körperlichen Belastung der überwiegende Teil der Muskelmasse des Probanden dynamisch und konstant belastet wird.

## Claims

1. Method of determining the general fitness of a subject comprising the following method steps:
Providing the qualitative temporal developing of the heart rate at a constant physical stress of a subject as a mathematical function with at least one parameter depending on a subject,
Measuring the heart rate of the subject at continuous physical stress during a time interval, wherein the subject undergoes a constant physical stress during this short time interval of less than 50 seconds and the heart rate of the subject is continuously measured,
Determining, with a computer device, the parameter or parameters of the mathematical function from the measurement values, and **characterized in**:
Determining, with the computer device, the maximum heart rate from the function and the measurement values by a numerical method, namely by extrapolation, and/or determining, with the computer device, the initial heart rate without physical stress from the function and the measurement values by a numerical method, namely by extrapolation.

2. Method according to claim 1, **characterized in that** additionally the power of the subject is measured during the physical stress.

3. Method according to claim 2, **characterized in that** the power is monitored during the time interval and kept constant within predetermined thresholds.

4. Method according to claim 3, **characterized in that** the subject is informed about keeping the constant power and/or about a deviation from the constant power.

5. Method according to one of the previous claims, **characterized in that** the endurance performance capacity is determined from the maximum heart rate and/or the initial heart rate and/or the slope characteristic of the heart rate.

6. Method according to one of the previous claims, **characterized in that** a training recommendation for the subject is determined from the maximum heart rate and/or the slope characteristic of the heart rate and/or the initial heart rate.

7. Method according to claim 5 or 6, **characterized in that** the physical characteristics of the subject are provided and considered in determining the endurance performance capacity and/or in determining a training recommendation.

8. Method according to one of the previous claims, **characterized in that** the parameters of the function are determined at each determined heart beat of the subject.

9. Method according to claim 8, **characterized in that** the measuring of the heart rate is finished if the parameters determined from at least two consecutive heart beats differ by less than a predetermined value.

10. Method according to one of the previous claims, **characterized in that** the qualitative temporal developing of the heart rate is determined as mathematical function from the temporal developing of the heart rate of a plurality of subjects under physical stress.

11. Method according to one of the previous claims, **characterized in that** during the physical stress the predominant portion of the muscle mass of the subject is dynamically and constantly stressed.

## Revendications

1. Procédé de détermination de la forme physique générale d'un sujet de test, comprenant les étapes de procédé suivantes :
prescription de la variation temporelle qualitative de la fréquence cardiaque lors d'un effort physique constant d'un sujet de test en tant que fonction mathématique avec au moins un paramètre dépendant d'un sujet de test, mesure de la fréquence cardiaque du sujet de test sous effort physique continu pendant un intervalle de temps, le sujet de test étant soumis pendant cet intervalle de temps court de moins de 50 secondes à un effort physique constant et, pendant cette durée, la fréquence cardiaque du sujet de test étant mesurée en continu,
détermination, avec un équipement de calcul, du ou des paramètres de la fonction mathématique à partir des valeurs mesurées,
et **caractérisé par** :
la détermination, avec l'équipement de calcul, de la fréquence cardiaque maximale à partir de la fonction et des valeurs mesurées par l'intermédiaire d'un procédé numérique, à savoir par extrapolation, et/ou la détermination, avec l'équipement de calcul, de la fréquence cardiaque initiale sans effort physique à partir de la fonction et des valeurs mesurées par l'intermédiaire d'un procédé numérique, à savoir par extrapolation.

2. Procédé selon la revendication 1, **caractérisé en ce qu'**en outre, la performance du sujet de test est déterminée pendant l'effort physique.

3. Procédé selon la revendication 2, **caractérisé en ce que** la performance est surveillée pendant l'intervalle de temps et maintenue constante dans des limites prescrites.

4. Procédé selon la revendication 3, **caractérisé en ce que** le sujet de test est informé sur le respect de la performance constante et/ou sur un écart par rapport à la performance constante.

5. Procédé selon une des revendications précédentes, **caractérisé en ce que** la capacité d'endurance est déterminée à partir de la fréquence cardiaque maximale et/ou de la fréquence cardiaque initiale et/ou de la caractéristique d'augmentation de la fréquence cardiaque.

6. Procédé selon une des revendications précédentes, **caractérisé en ce qu'**à partir de la fréquence cardiaque maximale et/ou de la caractéristique d'augmentation de la fréquence cardiaque et/ou de la fréquence cardiaque initiale est déterminée une recommandation d'entraînement pour le sujet de test.

7. Procédé selon la revendication 5 ou 6, **caractérisé en ce que** des caractéristiques physiques du sujet de test sont prescrites et sont prises en compte lors de la détermination de la capacité d'endurance et/ou lors de la détermination d'une recommandation d'entraînement.

8. Procédé selon une des revendications précédentes, **caractérisé en ce que** les paramètres de la fonction sont déterminés à chaque battement cardiaque enregistré du sujet de test.

9. Procédé selon la revendication 8, **caractérisé en ce que** la mesure de la fréquence cardiaque prend fin lorsque les paramètres déterminés à partir d'au moins deux battements cardiaques successifs diffèrent de moins une valeur prescrite.

10. Procédé selon une des revendications précédentes, **caractérisé en ce que** la variation temporelle qualitative de la fréquence cardiaque est déterminée comme fonction mathématique à partir de la variation temporelle de la fréquence cardiaque d'une pluralité de sujets de test sous effort physique.

11. Procédé selon une des revendications précédentes, **caractérisé en ce que**, pendant l'effort physique, la majeure partie de la masse musculaire du sujet de test est sollicitée de manière dynamique et constante.
